(19) Europäisches Patentamt
European Patent Office
Office européen des brevets

(11) Publication number: 0 300 972 A1

# (12) EUROPEAN PATENT APPLICATION

(21) Application number: 88810493.2

(22) Date of filing: 18.07.88

(51) Int. Cl.4: C 07 D 333/36
C 07 D 333/38, A 01 N 47/36

(30) Priority: 20.07.87 US 75504

(43) Date of publication of application:
25.01.89 Bulletin 89/04

(84) Designated Contracting States:
AT BE CH DE ES FR GB GR IT LI LU NL SE

(71) Applicant: SANDOZ AG
Lichtstrasse 35
CH-4002 Basel (CH)

(84) Designated Contracting States:
BE CH ES FR GB GR IT LI LU NL SE

(71) Applicant: SANDOZ-PATENT-GMBH
Humboldtstrasse 3
D-7850 Lörrach (DE)

(84) Designated Contracting States: DE

(71) Applicant: SANDOZ-ERFINDUNGEN
Verwaltungsgesellschaft m.b.H.
Brunner Strasse 59
A-1235 Wien (AT)

(84) Designated Contracting States: AT

(72) Inventor: Carney, Robert Leon
2676 Emerson Street
Palo Alto CA 94306 (US)

Gruber, John Myron
505 Cypress Point Drive 177
Mountain View CA 94043 (US)

Kuhnen, Fred
Reblistrasse 20
D-7858 Weil (DE)

Lui, Alfred Sui Ting
716 Windsor Way
Redwood City, CA 84062 (US)

The title of the invention has been amended (Guidelines for Examination in the EPO, A-III, 7.3).

(54) **Substituted N-benzoyl-N'-thienyl ureas, and their use as insecticides.**

(57) Substituted N-benzoyl-N'-thienyl-ureas, processes for their production and their use in the control of pests.

EP 0 300 972 A1

**Description**

## NOVEL COMPOUNDS

The present invention relates to substituted N-benzoyl-N'-thienyl-ureas, to processes for producing these compounds, to intermediates therefor and to the use of the compounds for the control of pests, and in particular for the control of insects and acarids.

More particularly the compounds of the present invention are represented by the following formula I:

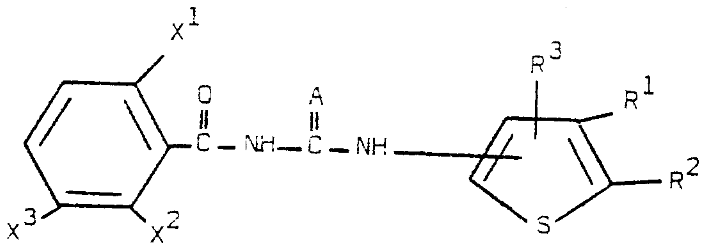

I

wherein,

A is oxygen or sulphur

each of $X^1$, $X^2$ and $X^3$ is independently hydrogen, $C_{1-8}$alkyl or halogen,

each of $R^1$ and $R^2$ is independently hydrogen, halogen, cyano, unsubstituted or halogenated $C_{1-8}$alkyl, or a group of formula II or III

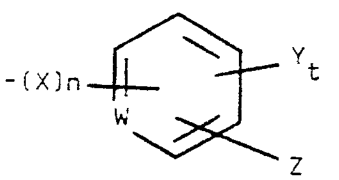

II

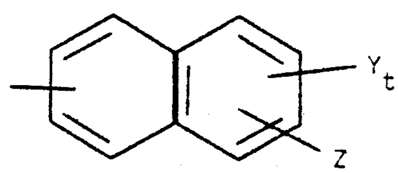

III

provided that when one of $R^1$ or $R^2$ is a group of formula II or III the other of $R^1$ or $R^2$ is not a group of formula II or III

$R^3$ is hydrogen, halogen or COOR

R is hydrogen or $C_{1-8}$alkyl

n is zero or one

t is one, two, three or four

W is nitrogen or CH

X is oxygen, sulphur or methylene

Y is hydrogen, halogen, or unsubstituted or halogenated $C_{1-8}$alkyl; and

Z is halogen, unsubstituted or halogenated $C_{1-8}$alkyl or unsubstituted or halogenated $C_{1-8}$alkoxy;

and agriculturally acceptable salts or metal complexes thereof.

Where any of the substituents $X^1$-$X^3$, $R^1$-$R^3$, Y and Z is or comprises halogen, such halogen is conveniently selected from bromo, chloro or fluoro.

Where any of $X^1$ to $X^3$, R-$R^2$, Y or Z is or comprises $C_{1-8}$alkyl, it is preferably of 1 to 4 carbon atoms and is more preferably of 1 or 2 carbons.

The terms halogenated $C_{1-8}$alkyl and halogenated $C_{1-8}$alkoxy refer to $C_{1-8}$alkyl and $C_{1-8}$alkoxy respectively, substituted by 1 to 8, preferably 1 to 6, and more preferably 1 to 3 halogens; such halogen is preferably chloro or fluoro and more preferably fluoro. Preferred examples of such substituents are trifluoromethyl and trifluoromethoxy.

Examples of suitable agriculturally acceptable salts or metal complexes of the acyl ureas are ammonium, sulfonium, phosphonium salts such as, for example, tetraethylammonium, benzyltrimethylammonium, trimethylsulfonium, trimethylsulfoxonium and ethyltriphenylphosphonium salts or titanium (IV), zirconium (IV) and zinc (II) complexes.

In the practice of the present invention;

$X^1$ is preferably halogen; such halogen preferably being chloro or fluoro.

$X^2$ is preferably hydrogen or halogen; such halogen preferably being fluoro.

$X^3$ is preferably hydrogen.

Each of $R^1$ and $R^2$, independently, conveniently signifies hydrogen, $C_{1-4}$alkyl, halogenated $C_{1-4}$alkyl, halogen or a group of formula II, preferably hydrogen, methyl, $CF_3$, chloro or a group of formula II, provided that when one of $R^1$ and $R^2$ is a group of formula II, the other of $R^1$ and $R^2$ is other than a group of formula II,

W is preferably CH
t is preferably one
X is preferably methylene or oxygen
Y is preferably hydrogen or halogen
Z is preferably hydrogen, unsubstituted or halogenated $C_{1-4}$alkyl, halogenated $C_{1-4}$alkoxy or halogen

The urea nitrogen atom is preferably bonded to the 2-position of the thiophene ring.

Particularly preferred compounds of formula I have one or more of the following features;
A is oxygen
$X^1$ is fluoro or chloro
$X^2$ is fluoro or hydrogen
$R^1$ and $R^2$ are independently hydrogen, $C_{1-4}$alkyl, chloro, trifluoromethyl or Y,Z-phenyl, more preferably $R^1$ is hydrogen, chloro, methyl or trifluoromethyl and $R^2$ is Y,Z-$C_6H_3$
$R^3$ is hydrogen, chloro or fluoro and most preferably hydrogen
n is 0
Y is hydrogen, chloro or fluoro and more preferably chloro or hydrogen and most preferably hydrogen
Z is chloro, bromo, fluoro, methyl, trifluoromethyl or trifluoromethoxy and most preferably bromo, chloro or trifluoromethyl.

The urea nitrogen is preferably bonded to the 2-position of the thienyl group and Z is preferably in the 4-position of the group of formula II.

The compounds of Formula I can be obtained by:
reacting a benzoyl isocyanate or isothiocyanate of formula IV

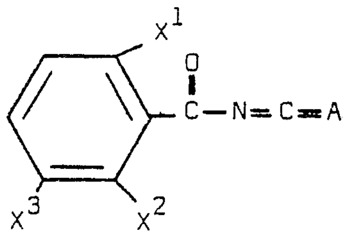

IV

wherein $X^1$, $X^2$, $X^3$ and A are as defined above, with an aminothiophene of formula V

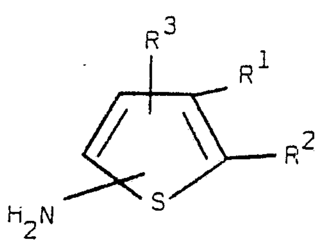

V

wherein $R^1$, $R^2$ and $R^3$ are as defined above, or the acid addition salt thereof.

The reaction of compounds of formula IV with compounds of formula V may be effected under the conditions known for the preparation of N-benzoyl-N′-ureas from the corresponding isocyanates and aminothiophenes or acid salts of aminothiophenes.

The reaction is conveniently carried out in a solvent which is inert under the reaction conditions, eg methylene chloride, toluene or dimethylformamide. A suitable reaction temperature may vary from -10° C to the boiling point of the solvent used, and preferably is about room temperature or moderately above or below room temperature, eg between 15° and 40° C. The reaction is optionally carried out in the presence of an organic base, for example triethylamine.

Salts of the compounds of formula I may be prepared by reaction of, for example, an ammonium, sulfonium or phosphonium halide with a compound of formula I in the presence of a base, such as, for example, potassium hydroxide, and a water-immiscible solvent, such as dichloromethane.

Metal complexes of the compounds of formula I may be prepared by, for example, reaction of a compound of formula I with a metal alkoxide, such as titanium tetraisopropoxide, under vacuum at a temperature that permits removal of the alcohol product by distillation, typically 75° to 100°.

The compounds of formula I may be recovered from the reaction mixture in which they are formed by working up by established procedures.

The starting materials and reagents employed in the processes described above are either known or, in cases where they are novel, may be produced by methods analogous to the processes described herein or to

known processes.

The compounds of formula IV can be synthesised by treating the corresponding benzamide with oxalyl chloride or by reacting the corresponding benzoyl chloride with ammonium thiocyanate.

The aminothiophene derivatives of formula V can be prepared by, for example,

a) reduction or catalytic hydrogenation of the corresponding nitro compounds,

b) for the preparation of 3-aminothiophenes, by the dehydration of the corresponding 2-$R^2$-3-$R^1$-4-oxotetrahydrophene oximes,

c) for the preparation of 2-aminothiophenes, by the reaction of the corresponding 4-$R^2$-4-oxobutyronitrile with hydrogen sulphide and hydrochloric acid or by reacting $R^2$-$CH_2$-CO-$R^1$ and t-butylcyanoacetate with sulphur in the presence of diethylamine followed by acid hydrolysis.

The compounds of formula I are chitin inhibitors as indicated by tests with i.a. third instar larvae of Manduca sexta, Musca domestica, Heliothis vireseens and Spodoptera exigua, fourth instar larvae of Aedes aegypti and first instar larvae of Dermestes maculatus. They are accordingly indicated for use as pest controlling agents, particularly for the control of insects, mites and ticks.

In view of their interesting activity, particularly with regard to the level and spectrum of activity, the compounds of formula I offer an advantageous alternative to known chitin inhibitors, such as those described in US Patent 3,748,356 and UK Patent 2,134,518.

The compounds of formula I can be effective control agents for insects of, for example, the orders Lepidoptera, Hemiptera, Homoptera, Coleoptera, Diptera, Orthoptera and Siphonaptera, and other insects, as well as for mites and ticks of the class Acari, including mites of the families Tetranychidae and Tarsonemidae and ticks of the families Argasidae and Ixodidae. The compounds can be applied to the pest or its locus in a pest controlling amount, usually of the order of 0.001 µg to 100 µg, preferably 0.001 µg to 0.1 µg, per insect, mite or tick, depending on the mode and conditions of application as well as on the pest involved.

The compounds may be useful anthelminthic agents against parasites of warm-blooded animals and of plants, such as, for example, intestinal and extraintestinal nematodes of the families Ascaridae and Tricho strongylidae and plant parasitic nematodes of the families Heteroderidae and Tylenchidae.

Additionally, compounds of formula I may possess a repellant and/or antifeedant action on terrestrial snails and slugs. The compounds may also be useful for control of arthropod endoparasites and ectoparasites of vertebrates, either by topical application or by oral administration to the host animal.

The compounds of the present invention may be used as an active ingredient in anti-fouling marine paints to prevent attachment of marine anthropods such as barnacles.

In the use of the compounds of formula I for combatting pests, a compound of formula I, or mixtures therof, can conveniently be employed as a pesticidal composition in association with acceptable diluent(s) for application to the pest or its locus. Such compositions also form part of the present invention.

Suitable formulations contain from 0.01 to 99% by weight of active ingredient, from 0 to 20% of surfactant and from 1 to 99.99% of solid or liquid diluent(s). Higher ratios of surfactant to active ingredient are sometimes desirable and are achieved by incorporation into the formulation or by tank mixing. Application forms of a composition generally contain between 0.01 and 25% by weight of active ingredient. Lower or higher levels of active ingredient can, of course, be present depending on the intended use, the physical properties of the compound and the mode of application. Concentrate forms of a composition intended to be diluted before use generally contain between 2 and 90%, preferably between 5 and 80 % by weight of active ingredients.

Useful formulations of the compounds of formula I include dusts, granules, suspension concentrates, wettable powders, flowables and the like. They are obtained by conventional manner, eg by mixing a compound of formula I with the diluent(s) and optionally with other ingredients.

Alternatively, the compounds of formula I may be used in microencapsulated form.

The compounds of formula I can be combined with beta or gamma cyclodextrin to make a cyclodextrin inclusion complex for application to the pest or its locus.

Agriculturally acceptable additives may be employed in the pesticidal compositions to improve the performance of the active ingredient and to reduce foaming, caking and corrosion, for example.

"Surfactant" as used herein means an agriculturally acceptable material which imparts emulsifiability, spreading, wetting, dispersibility or other surface-modifying properties. Examples of surfactants are sodium lignin sulfonate and lauryl sulfate.

"Diluent" as used herein means a liquid or solid agriculturally acceptable material used to dilute a concentrated material to a usable or desirable strength. For dusts or granules it can be eg talc, kaolin or diatomaceous earth, for liquid concentrate forms for example a hydrocarbon such as xylene or an alcohol such as isopropanol, and for liquid application forms ie water or diesel oil.

The compositions of this invention can also comprise other compounds having biological activity, eg compounds having similar or complementary pesticidal or insect growth regulating activity or compounds having antidotal, fungicidal, herbicidal or insect attractant activity.

The following examples are provided to illustrate the practice of the present ivention. Temperature is given in degrees Centigrade. RT means room temperature. Parts and percentages are by weight.

COMPOSITION EXAMPLES

**Example A:** Flowable

| | |
|---|---|
| Compound 1 | 26.00% |
| dispersant | 5.20% |
| thickener | 0.58% |
| antifoam | 0.10% |
| water | 61.52% |
| propylene glycol | 6.60% |

**Example B:** Wettable Powder

| | |
|---|---|
| Compound 1 | 81.0% |
| kaolin | 14.8% |
| disperant | 4.0% |
| wetting agent | 0.2% |

## PREPARATION OF FINAL UREAS

**Example 1:** N-2,6-difluorobenzoyl-N-[5-(4-chlorophenyl)-4-methyl-2-thienyl]-urea

To a solution of 2-amino-4-methyl-5-(5-chlorophenyl)-thiophene HCl (0.45g, 1.73mmol) in 4ml of dimethylformamide and 4ml of methylene chloride is added 2,6-difluorobenzoylisocyanate (0.32g, 1.73mmol), followed by triethylamine (0.23g, 0.3ml, 2.25mmol). The mixture is stirred overnight at RT, after which water is added and the solvent is removed. The residue is filtered, and the solid is washed with 50% hexane/methylene chloride to give the title product. (Table A, Compound 1)

**Example 2:** N-2,6-difluorobenzoyl-N′-[5-(4-trifluoromethoxyphenyl)-4-methyl-2-thienyl]urea

To a solution of 2-amino-4-methyl-5-(4-trifluoromethoxyphenyl)thiophene trifluoroacetic acid salt (1.88mmol) in 30ml of methylene chloride is added, with cooling, triethylamine (3.75mmol), followed by 2,6-difluorobenzoylisocyanate (1.88mmol). The mixture is worked up as in Example 1 to give the title compound (Table A, Compound 41)

**Example 3:**

Following the procedures of Example 1, each of the final compounds 2-24, 36-40, 42-45 and 50-51 under Table A and 25-31 under Table B is prepared from the corresponding thiophene or thiophene acid salt and benzoyl isocyanate or isothiocyanate intermediates.

**Example 4**

3-amino-5-(4-chlorophenyl)-thiophene is reacted with each of 2,6-difluorobenzoyl isocyanate and 2-chlorobenzoyl isocyanate, following the procedure of Example 1 to give, respectively;

i) N-2,6-difluorobenzoyl-N-[5-(4-chlorophenyl)-3-thienyl]-urea (Table C, Compound 32)

ii) N-2-chlorobenzoyl-N-[5-(4-chlorophenyl)-3-thienyl]-urea (Table C, Compound 33)

In the same manner each of the final compounds under Table C is prepared from the corresponding thiophene or thiophene acid salt and benzoyl isocyanate or isothiocyanate intermediates.

## TABLE A

Compounds of formula I in which $X^3 = H$, $R^2$ is a group of formula II, W is CH, t = 1 and the urea nitrogen is bonded to the 2-position of the thienyl group

| CPD | $X^1$ | $X^2$ | A | $R^1$ | $R^3$ | Y | Z | n | X | mp |
|---|---|---|---|---|---|---|---|---|---|---|
| 1 | F | F | O | $CH_3$ | H | H | 4-Cl | 0 | - | 203-205° |
| 2 | Cl | H | O | $CH_3$ | H | H | 4-Cl | 0 | - | 188-190° |
| 3 | F | H | O | $CH_3$ | H | H | 4-Cl | 0 | - | 186-188° |
| 4 | F | F | O | $CF_3$ | H | H | 4-Cl | 0 | - | 208-209° |
| 5 | F | F | O | H | H | H | 4-Cl | 0 | - | 231-232° |
| 6 | Cl | H | O | H | H | H | 4-Cl | 0 | - | 216-218° |
| 7 | F | F | O | H | H | 3-Cl | 4-Cl | 0 | - | 211-213° |
| 8 | Cl | H | O | H | H | 3-Cl | 4-Cl | 0 | - | 201-202° |
| 9 | F | F | O | $CH_3$ | H | H | 4-F | 0 | - | 188-190° |
| 10 | Cl | H | O | $CH_3$ | H | H | 4-F | 0 | - | 175-177° |
| 11 | F | F | O | $CH_3$ | H | H | 4-Br | 0 | - | 207-209° |
| 12 | Cl | H | O | $CH_3$ | H | H | 4-Br | 0 | - | 197-199° |
| 13 | F | F | O | $CH_3$ | H | H | 4-$CF_3$ | 0 | - | 228-229° |
| 14 | Cl | H | O | $CH_3$ | H | H | 4-$CF_3$ | 0 | - | 202-204° |
| 15 | F | F | O | $CH_3$ | H | 3-Cl | 4-Cl | 0 | - | 181-183° |
| 16 | Cl | H | O | $CH_3$ | H | 3-Cl | 4-Cl | 0 | - | 195-197° |
| 17 | F | F | O | $CH_3$ | H | H | 4-H | 0 | - | 203-205° |
| 18 | Cl | H | O | $CH_3$ | H | H | 4-H | 0 | - | 207-208° |
| 19 | F | F | O | Cl | H | H | 4-Cl | 0 | - | 211-213° |
| 20 | Cl | H | O | Cl | H | H | 4-Cl | 0 | - | 201-202° |
| 21 | Cl | H | O | H | Cl | H | 4-Cl | 0 | - | 243-245° |
| 22 | Cl | H | O | Cl | Cl | H | 4-Cl | 0 | - | oil |
| 23 | Cl | H | O | $CH_3$ | COOH | H | 4-Cl | 0 | - | 235° |
| 24 | F | F | O | $CH_3$ | COOH | H | 4-Cl | 0 | - | 240-241° |
| 36 | F | F | S | $CH_3$ | H | H | 4-Cl | 0 | - | 180.5-181.5 |

| CPD | $X^1$ | $X^2$ | A | $R^1$ | $R^3$ | Y | Z | n | X | mp |
|---|---|---|---|---|---|---|---|---|---|---|
| 37 | F | F | O | $CH_3$ | $COOCH_3$ | H | 4-Cl | 0 | - | 275-278° |
| 38 | Cl | H | O | $CH_3$ | $COOCH_3$ | H | 4-Cl | 0 | - | 245-246° |
| 39 | F | F | O | $CH_3$ | $COOC(CH_3)_3$ | H | 4-Cl | 0 | - | 207-208° |
| 40 | Cl | F | O | $CH_3$ | $COOC(CH_3)_3$ | H | 4-Cl | 0 | - | 243-244° |
| 41 | F | F | O | $CH_3$ | H | H | 4-$OCF_3$ | 0 | - | 201-202° |
| 42 | F | F | O | $CH_3$ | H | 2-Cl | 4-$CF_3$ | 0 | - | 199-200° |
| 43 | F | F | O | $CH_3$ | H | 2-F | 4-Cl | 0 | - | |
| 44 | F | F | O | $CH_3$ | H | 3-Cl | 5-Cl | 0 | - | 239-240° |
| 45 | F | F | O | $CH_3$ | H | 2-Cl | 5-$CF_3$ | 0 | - | |
| 50 | F | F | O | $CH_3$ | H | H | 4-Cl | 1 | O | 202-203° |
| 51 | F | F | O | $CH_3$ | H | 2-Cl | 4-$OCF_3$ | 0 | - | 171-172° |

TABLE B

Compounds of formula I in which $X^3$ = H, $R^3$ = H and the urea nitrogen is bonded to the 2-position of the thienyl ring.

| CPD | $X^1$ | $X^2$ | A | $R^1$ | $R^2$ | mp |
|---|---|---|---|---|---|---|
| 25 | F | F | 0 | Cl | Cl | 215-219° |
| 26 | Cl | H | 0 | Cl | Cl | 225-227° |
| 27 | F | F | 0 | 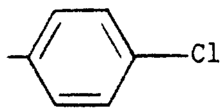 | H | 247-249° |
| 28 | Cl | H | 0 | 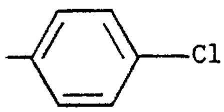 | H | 217-219° |
| 29 | F | F | 0 | 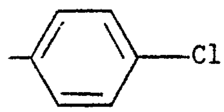 | $CH_3$ | 230-231° |
| 30 | Cl | H | 0 | 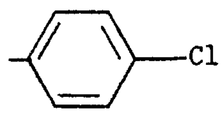 | $CH_3$ | |
| 31 | F | F | 0 | $-(CH_2)-$ 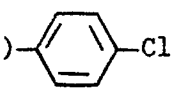 | H | 194-196° |

TABLE C

Compounds of formula I in which $X^3$ = H and the urea nitrogen is bonded to the 2-position of the thienyl ring.

| Cpd | $X^1$ | $X^2$ | A | $R^1$ | $R^2$ | $R^3$ | mp |
|---|---|---|---|---|---|---|---|
| 32 | F | F | O | H | 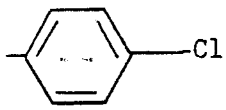 | H | 235-237° |
| 33 | Cl | H | O | H | 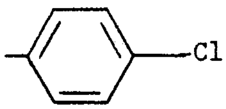 | H | 214-216° |
| 34 | F | F | O | H | 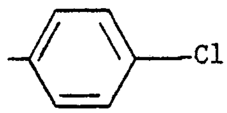 | Cl | 243-245° |
| 35 | Cl | H | O | H | 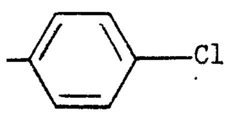 | Cl | 220° |
| 46 | F | H | O | H | $CF_3$ | H | 134° |
| 47 | Cl | H | O | H | $CF_3$ | H | 177° |
| 48 | F | F | O | H | $CF_3$ | H | 180-181° |
| 49 | Cl | Cl | O | H | $CF_3$ | H | 182-184° |

## PREPARATION OF INTERMEDIATE COMPOUNDS

The following examples are presented to illustrate representative methods of preparing the intermediate aminothiophene hydrochloric acid or trifluoroacetic acid salts.

**Example 5:** 2-amino-4-methyl-5-(4-chlorophenyl)thiophene HCl

To a mixture of 4-chlorophenylacetone (5.0g, 29.7mmol), t-butyl cyanoacetate (4.19g, 29.7mmol) in 12ml of ethanol is added diethylamine (4ml), and the mixture is heated to 60° for 4 hours. The reaction mixture is then diluted with water and extracted with ethyl acetate. The combined organic layers are washed with water and brine and dried, and the crude product is purified to give 2-amino-3-(t-butoxycarbonyl)-4-methyl-5-(4-chlorophenyl)thiophene.

The above thiophene (2.0g) in 10ml of 6M HCl is heated at 80° for 33 hours. Ethanol is added, and the solvent and water are then removed. Ether is added to the residue, the suspension is filtered and the final solid is rinsed with ether to give 2-amino-4-methyl-5-(4-chlorophenyl)thiophene hydrochloric acid salt.

**Example 6:** 2-amino-4-trifluoromethyl-5-(4-chlorophenyl)-thiophene trifluroracetic acid salt

Following the procedure of Example 4, 2-amino-3-(t-butylcarbonyl)-4-trifluoromethyl-5-(4-chlorophenyl)thiophene is prepared from 1,1,1-trifluoro-3-(4-chlorophenyl)acetone (2.60g), t-butylcyanoacetate (1.65g) and sulfur (0.37g).

The above thiophene (0.30g) is treated with trifluoroacetic acid (TFA) (3ml), and the mixture is stirred at RT overnight. The excess TFA is evaporated off to give the title product.

**Example 7:** 2-amino-4-(4-chlorophenyl)-5-methylthiophene trifluoroacetic acid salt

A stirred mixture of 4-chloropropiophenone (4.0g, 24.0mmol) and t-butylcyanoacetate (4.0g, 4,1ml, 28.0mmol) in 40 ml of toluene and 2ml of acetic acid is heated under reflux. Ammonium acetate (1.82g, 24.0mmol) is added in portions over 72 hours as the water formed in the reaction is removed azeotropically. The reaction is then cooled, stripped of solvents, and the resulting clear oil mixture purified by chromatography to yield (E,Z) t-butyl-3-(4-chlorophenyl)-2-cyano-2-pentanoate.

A mixture of the above pentanoate (1.5g, 5.1mmol), sulfur (0.18g, 5.7mmol), ethanol (15ml) and diethylamine (7ml) is stirred at 40° C for 3 hours. The reaction mixture is taken up in ether and washed with water and brine, then dried over sodium sulfate and the solvents stripped off. The product is purified by chromatography to yield 2-amino-3-(t-butoxycarbonyl)-4-(4-chlorophenyl)-5-methylthiophene.

The above thiophene (0.70g) is stirred for 20 hours trifluoroacetic acid is evaporated off to yield.

**Example 8:** 2-amino-5-(4-chlorophenyl)thiophene HCl

To a mixture of freshly ground sodium cyanide (0.75g, 15.0mmol) in dimethylformamide (DMF) is added over 30 mins, 4-chlorobenzaldehyde (21.0g, 150.0mmol) in DMF. 30 mins after the addition is completed, acrylonitrile (5.96g, 7.4ml, 112.5mmol) is added over a period of one hour. The mixture is stirred at RT for 3 hours, after which acetic acid (0.99g, 0.94ml, 16.5mmol) is added. After 5 mins, the reaction mixture is poured into water and extracted with chloroform. The combined chloroform layers are washed with water and with brine, dried and stripped of solvent. The residue is recrystallised and filtered to give 4-(4-chlorophenyl)-4-oxo-butyronitrile.

HCl and $H_2S$ gas are bubbled through a solution of the above nitrile (7.70g, 40.0mmol) in 100ml of methanol at 10°-20° for 7 hours. The suspension is then stirred at RT overnight. It is dried, filtered, washed with ether and dried with suction under an $N_2$ atmosphere to give the title product as a slightly purplish powder.

**Example 9:** 2-amino-4,5-dichlorothiophene HCl

To a solution of 2-nitrothiopnene (10.0g, 77.5mmol) in 60ml of chloroform is added aluminium chloride (6.0g, 45.0mmol). The mixture is colled to 10° and chloride gas is bubbled through for 3 hours at 25-30°. Nitrogen gas is then bubbled through the mixture of r 10 mins, after which the mixture is poured into ice water and extracted with chloroform. The combined chloroform layers are washed with water and with brine and dried to give 2-nitro-4,5-dichlorothiophene.

To a cooled solution of the above 4,5-dichlorothiophene (13.0g, 66.0mmol) are added acetic acid (78ml) and acetic anhydrid (78ml), followed by iron powder (13.0g) in portions over 30 mins at such rate that the mixture is kept at 5°-10°. The suspension is stirred at RT overnight, after which it is poured into water, acidified with conc HCl and cooled in an ice bath for 6 hours. It is then filtered, and the solid is washed with water and dired, dissoved in ether and filtered. Charcoal is added to the filtrate, which is then filtered and stripped of solvent to give 4,5-dichloro-2-thienylacetamide, a solid.

HCl gas is bubbled through a solution of the above amide (1.5g) in 10ml of methanol for one hour. The resulting suspension is stripped of solvent, and the residue is filtered and washed with ether to give 2-amino-4,5-dichlorothiophene hydrochloric acid salt.

**Example 10:** 2-amino-4-chloro-5-(4-chlorophenyl)thiophene HCl

4-chloroaniline (4.0g, 31.4mmol) is addd to 4ml of acetic acid and heated until all solid is dissolved. The solution is cooled to 5° and 5.2ml of conc HCl is added, followed by the dropwise addition of sodium nitrite (2.16g, 31.4mmol) in 32ml water. After addition is complete, the mixture is stirred at 5° for one hour. Thiophene (2.64g, 2.5ml, 31.4mmol) in 25ml of carbone tetrachloride is added to the mixture, followed by the dropwise addition of 25% aq NaOH (ca 6.8ml). After one hour, the mixture is poured into water and extracted with chloroform, and the combined extracts are washed with brine, dried and stripped of solvent. The black residue is distilled at ca 120° 0.35mm to give 2-(4-chlorophenyl)thiophene, a yellow solid.

A solution of the above phenylthiophene (10.6g, 54.0mmol) in 100ml of acetic anhydride is added dropwise to a solution of cupric nitrate (6.3g, 27.0mmol) in 100ml of acetic anhydride at 10°-12°. The mixture is stirred at Rt for 2 hours, then poured into ice water and extracted with ethyl acetate. the combined organic layers are wasjed wotj water amd with brine, dried and stripped of solvent to give a dark brown solid which is purified to yield 2-nitro-5-(4-chlorophenyl)thiophene.

Chlorine gas is bubbled through a suspension of the above nitrothiophene (0.5g) in 6ml of acetic acid for 3.5 hours, after which the solvent is stripped off and the residue is purified to give 2-nitro-4-chloro-5-(4-chlorophenyl)thiophene.

Following the procedure of Example 8, 2-nitro-4-chloro-5-(4-chlorophenyl)thiophene (2.3g) is reacted with acetic anhydride (14ml) and iron powder (2.3g) in acetic acid (14ml), followed by reaction with HCl and methanol (30ml) to give the title compound.

**Example 11:** 3-amino-5-(4-chlorophenyl)thiophene HCl

To a stirred mixture of mercaptoacetic acid (5.04g, 3.8ml, 54.7mmol) and 4-chlorocinnamic acid (10.0g, 54.7mmol) in 20ml of dioxane is added dropwise a solution of triethylamine (6.90g, 9.53ml, 68.0mmol) in 20ml dioxane. The mixture is heated under reflux for 5 hours, then cooled and poured into ice, acidified with conc sulfuric acid and extracted with ether. The combined ether extracts are washed with water and with brine, dried and stripped of solvent. The residue is washed with chloroform to give 3-carboxymethylthio-3-(4-chlorophenyl)propionic acid, a yellow solid.

A mixture of the above propionic acid (12.75g, 46.6mmol), lithium carbonate (0.23g) and acetic anhydride (35ml) is heated under reflux overnight. The mixture is then poured into ice and conc sulfuric acid and stirred at under 25° for 2 hours. It is extracted with ether, and the ether extracts are stripped of solvent. The residue is taken up in ether and absified, then washed with brine, dried, stripped of solvent and purified to give 5-(4-chlorophenyl)-3-oxotetrahydrothiophene, a yellow solid.

A mixture of the above tetrahydrothiophene (5.70g,27.0mmol) hydroxylamine HCl (2.81g, 40.0mmol) and barium carbonate (5.60g) in 70ml of ethanol is heated under reflux overnight. The solid is filtered and washed with 250ml of hot ethanol. The solvent is stripped off, and the residue is diluted with ether and ethyl acetate, washed with water and with brine, dried and stripped of solvent to give 2-(4-chlorophenyl)-4-oxotetrahydrothiophene oxime.

Following the procedure of Example 8, the above oxime (6.14g, 27.0mmol) is reacted with HCl and methanol to give 3-amino-5-(4-chlorophenyl)thiophene hydrochloric acid salt.

## Claims

1 A compound of formula I

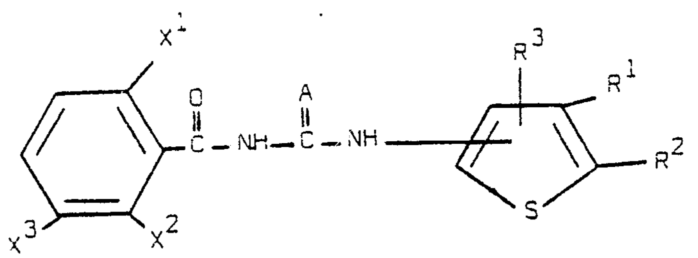

I

wherein,
A is oxygen or sulphur
each of $X^1$, $X^2$ and $X^3$ is independently hydrogen, $C_{1-8}$alkyl or halogen,
each or $R^1$ and $R^2$ is independently hydrogen, halogen, cyano, nitro, unsubstituted or halogenated $C_{1-8}$alkyl or a group of formula II or III

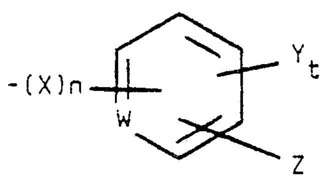

II

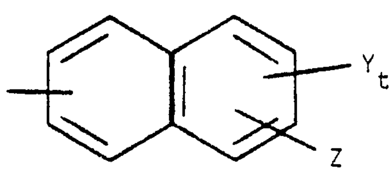

III

provided that when one of $R^1$ or $R^2$ is a group of formula II or III the other of $R^1$ or $R^2$ is not a group of formula II or III
$R^3$ is hydrogen, halogen or COOR
R is hydrogen or $C_{1-8}$alkyl
n is zero or one
t is one, two, three or four
W is nitrogen or CH
X is oxygen, sulphur or methylene
Y is hydrogen, halogen or unsubstituted or halogenated $C_{1-8}$alkyl; and
Z is halogen, unsubstituted or halogenated $C_{1-8}$alkyl or unsubstituted or halogenated $C_{1-8}$alkoxy;
and agriculturally acceptable salts or metal complexes thereof.

2 A compound according to claim 1 wherein;
$X^1$ is halogen
$X^2$ is hydrogen or halogen
$X^3$ is hydrogen
$R^1$ and $R^2$ are independently hydrogen, halogen, $C_{1-4}$alkyl, halogenated $C_{1-4}$alkyl or a group of formula II as defined in claim 1 with the proviso that when one of $R^1$ and $R^2$ is a group of formula II the other of $R^1$ and $R^2$ is other than a group of formula II
R is hydrogen or $C_{1-4}$alkyl
t is one
W is CH
X is methylene
Y is hydrogen or halogen
Z is halogen, unsubstituted or halogenated $C_{1-4}$alkyl or halogenated $C_{1-4}$alkoxy.

3 A compound according to claim 2 wherein A is oxygen, $R^3$ is hydrogen, chloro or fluoro, n is zero, Z is chloro, fluoro, bromo, methyl, trifluoromethyl or trifluoromethoxy and $R^1$ is hydrogen, chloro, methyl, trifluoromethyl or a group of formula II.

4 A compound according to claim 3 wherein the urea nitrogen is bonded to the 2 position of the thienyl ring.

5 A compound according to claim 4 wherein Y is hydrogen, Z is bromo, chloro or trifluoromethyl and $R^1$ is methyl.

6 A compound according to claim 5 wherein $X^1$ is chloro and $X^2$ is hydrogen.

7 A compound according to claim 4 wherein each of $X^1$ and $X^2$ is fluoro.

8 A compound according to claim 1 selected from the group consisting of:

a) N-2,6-difluorobenzoyl-N'-[5-(4-chlorophenyl-4-methyl-2-thienyl]urea.
b) N-2,6-difluorobenzoyl-N'-[4-methyl-5-(4-trifluoromethylphenyl)-2-thienyl]urea,
c) N-2,6-difluorobenzoyl-N'-[4-methyl-5-(4-bromophenyl)-2-thienyl]urea,
d) N-2,6-difluorobenzoyl-N'-[4-methyl-5-(2,4-dichlorophenyl)-2-thienyl]urea.

9 A compound according to claim 7 wherein Y is in the 2- or 3-position and is chloro or fluoro and Z is in the 4- or 5- position and is chloro, methyl or trifluoromethyl.

10 A process for the production of compounds of formula I which comprises reacting a benzoyl isocyanate or isothiocyanate of formula IV

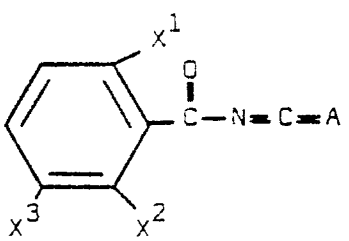

IV

wherein $X^1$ to $X^3$ and A are as defined in claim 1, with an aminothiophene of formula V

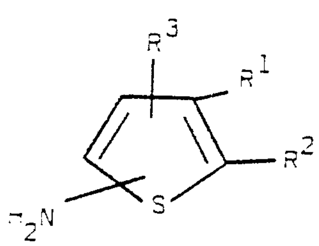

V

wherein $R^1$ to $R^3$ are as defined in claim 1, or the acid addition salt thereof.

11 A method for the control of pests which comprises applying to the pest or its locus a pest controlling amount of a compound of formula I as defined in any one of claims 1 to 9.

12 An insecticidal composition comprising an insecticidal amount of a compound of formula I as defined in any one of claims 1 to 9.

**Claims for the following contracting State: ES**

1 A process for the preparation of a compound of formula I

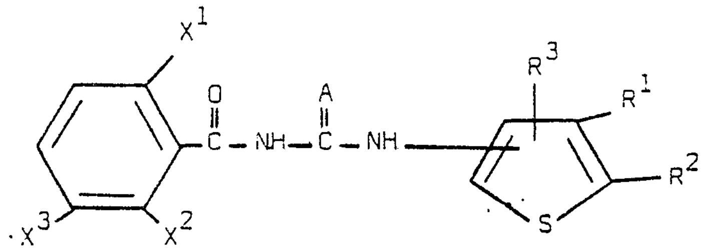

I

wherein,

A is oxygen or sulphur

each of $X^1$, $X^2$ and $X^3$ is independently hydrogen, $C_{1-8}$alkyl or halogen,

each of $R^1$ and $R^2$ is independently hydrogen, halogen, cyano, nitro, unsubstituted or halogenated $C_{1-8}$alkyl or a group of formula II or III

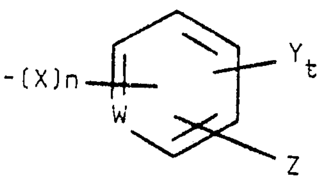

II

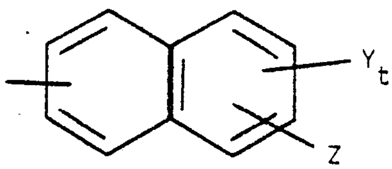

III

provided that when one of $R^1$ or $R^2$ is a group of formula II or III the other of $R^1$ or $R^2$ is not a group of formula II or III

$R^3$ is hydrogen, halogen or COOR

R is hydrogen or $C_{1-8}$alkyl

n is zero or one

t is one, two, three or four

W is nitrogen or CH

X is oxygen, sulphur or methylene

Y is hydrogen, halogen or unsubstituted or halogenated $C_{1-8}$alkyl; and

Z is halogen, unsubstituted or halogenated $C_{1-8}$alkyl or unsubstituted or halogenated $C_{1-8}$alkoxy;

and agriculturally acceptable salts or metal complexes thereof, which comprises reacting a benzoyl isocyanate or isothiocyanate of formula IV

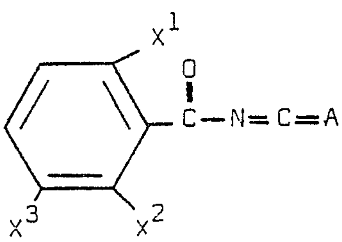

IV

wherein $X^1$ to $X^3$ and A are as defined above, with an aminothiophene of formula V

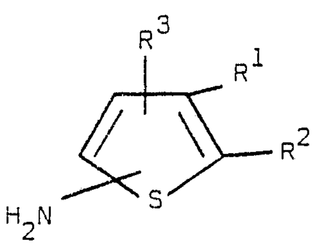

V

wherein $R^1$ to $R^3$ are as defined above, or the acid addition salt thereof.

2 A process according to claim 1 wherein;
$X^1$ is halogen
$X^2$ is hydrogen or halogen
$X^3$ is hydrogen
$R^1$ and $R^2$ are independently hydrogen, halogen, $C_{1-4}$alkyl, halogenated $C_{1-4}$alkyl or a group of formula II as defined in claim 1 with the proviso that when one of $R^1$ and $R^2$ is a group of formula II the other of $R^1$ and $R^2$ is other than a group of formula II
R is hydrogen or $C_{1-4}$alkyl
t is one
W is CH
X is methylene
Y is hydrogen or halogen
Z is halogen, unsubstituted or halogenated $C_{1-4}$alkyl or halogenated $C_{1-4}$alkoxy.

3 A process according to claim 2 wherein A is oxygen, $R^3$ is hydrogen, chloro or fluoro, n is zero, Z is chloro, fluoro, bromo, methyl, trifluoromethyl or trifluoromethoxy and $R^1$ is hydrogen, chloro, methyl, trifluoromethyl or a group of formula II.

4 A process according to claim 3 wherein the urea nitrogen is bonded to the 2 position of the thienyl ring.

5 A process according to claim 4 wherein Y is hydrogen, Z is bromo, chloro or trifluoromethyl and $R^1$ is methyl.

6 A process according to claim 5 wherein $X^1$ is chloro and $X^2$ is hydrogen.

7 A process according to claim 4 wherein each of $X^1$ and $X^2$ is fluoro.

8 A process according to claim 7 wherein Y is in the 2- or 3- position and is chloro or fluoro and Z is in the 4- or 5- position and is chloro, methyl or trifluoromethyl.

9 A process according to claim 1 wherein A is oxygen, $R^1$ is 4-methyl, $R^3$ is hydrogen, X1 is fluoro, $X^2$ is fluoro, $X^3$ is hydrogen and

a) $R^2$ is 5-(4-chlorophenyl)
b) $R^2$ is 5-(4-trifluoromethylphenyl)
c) $R^2$ is 5-(4-bromophenyl)
d) $R^2$ is 5-(2,4-dichlorophenyl).

10 An insecticidal composition comprising a compound of formula I as defined in any one of claims 1 to 9 and an agriculturally acceptable diluent.

11 Concentrate forms of a composition of claim 10 comprising from 5 to 85% by weight of a compound of formula I.

12 A process according to any one of claims 1 to 9 substantially as herein described by way of example.

European Patent Office

# EUROPEAN SEARCH REPORT

Application Number

EP 88 81 0493

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 82, no. 25, 23rd June 1975, page 556, no. 171034m, Columbus, Ohio, US; & SU-A-455 105 (UZHGOROD STATE UNIVERSITY) 30-12-1974 * Abstract, especially formula II * | 1 | C 07 D 333/36<br>C 07 D 333/38<br>A 01 N 47/36 |
| A | US-A-4 599 356 (LANGE et al.) * Column 1, lines 54-67; column 2, lines 1-10; claims * | 1,10-12 | |
| D,A | US-A-3 748 356 (K.WELLINGA) * Columns 1,2,11 * | 1,10-12 | |
| | | | **TECHNICAL FIELDS SEARCHED (Int. Cl.4)** |
| | | | C 07 D 333/00<br>C 07 C 127/00<br>A 01 N 47/00 |

The present search report has been drawn up for all claims

| Place of search | Date of completion of the search | Examiner |
|---|---|---|
| THE HAGUE | 11-10-1988 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document